# EUROPÄISCHE PATENTSCHRIFT

(11) **EP 3 394 610 B1**
(45) Veröffentlichungstag und Bekanntmachung des Hinweises auf die Patenterteilung: **01.02.2023**
(21) Anmeldenummer: 16823288.2
(22) Anmeldetag: 22.12.2016
(51) Int. Cl.: G01N 33/08, A01K 43/00, A01K 45/00

(54) **VERFAHREN ZUR IN OVO GESCHLECHTERBESTIMMUNG VON KÜKEN**
METHOD FOR THE IN-OVO SEX IDENTIFICATION OF CHICKS
PROCÉDÉ DE SEXAGE IN OVO DE POUSSINS

(30) Priorität: 22.12.2015 DE 102015226490
(43) Veröffentlichungstag der Anmeldung: 31.10.2018
(73) Patentinhaber: OVONDO GmbH, 04288 Leipzig (DE)
(72) Erfinder: EINSPANIER, Almuth, 04103 Leipzig (DE)
(74) Vertreter: Grünecker Patent- und Rechtsanwälte PartG mbB
(86) Internationale Anmeldenummer: PCT/EP2016/082483
(87) Internationale Veröffentlichungsnummer: WO 2017/109133

(56) Entgegenhaltungen:
- WO-A2-2006/124456
- US-A- 5 622 871
- US-A- 5 830 767
- US-A1- 2012 225 438
- US-A1- 2012 282 636
- US-A1- 2015 337 372
- A. WEISSMANN ET AL: "Sexing domestic chicken before hatch: A new method for in ovo gender identification", THERIOGENOLOGY, Bd. 80, Nr. 3, August 2013 (2013-08), Seiten 199-205, XP055348708, US ISSN: 0093-691X, DOI: 10.1016/j.theriogenology.2013.04.014
- LAITINEN M P A ET AL: "IMMUNOCHROMATOGRAPHIC ASSAY FOR QUANTITATION OF MILK PROGESTERONE", ACTA CHEMICA SCANDINAVICA, MUNKSGAARD, COPENHAGEN, DK, vol. 50, no. 2, 1 February 1996 (1996-02-01), pages 141-145, XP001108911, ISSN: 0904-213X
- MANICKUM THAVRIN ET AL: "The current preference for the immuno-analytical ELISA method for quantitation of steroid hormones (endocrine disruptor compounds) in wastewater in South Africa", ANALYTICAL AND BIOANALYTICAL CHEMISTRY, SPRINGER, DE, vol. 407, no. 17, 7 April 2015 (2015-04-07), pages 4949-4970, XP035487887, ISSN: 1618-2642, DOI: 10.1007/S00216-015-8546-0 [retrieved on 2015-04-07]
- Naoki Isobe ET AL: "Effects of Estrone Sulfate Administration on Reproductive Functions in Male Japanese Quail", JOURNAL OF POULTRY SCIENCE, vol. 40, no. 4, 1 January 2003 (2003-01-01), pages 247-253, XP055658404, JP ISSN: 1346-7395, DOI: 10.2141/jpsa.40.247

## Beschreibung

Die vorliegende Erfindung betrifft ein Verfahren zur in ovo Geschlechterbestimmung von Küken. Ein gattungsgemäßes Verfahren ist beispielsweise aus der Dissertation von Dr. Anne Weissmann, Universität Leipzig, aus dem Jahre 2014 mit dem Titel "in-ovo-Geschlechterbestimmung bei Legehybriden mittels endokriner Analyse der Allantoisflüssigkeit" bekannt.

A. WEISSMANN ET AL: "Sexing domestic chicken before hatch: A new method for in ovo gender identification",THERIOGENOLOGY, Bd. 80, Nr. 3, August 2013 (2013-08), Seiten 199-205 offenbart ebenfalls ein solches gattungsgemäßes Verfahren.

Zurzeit werden jährlich in Deutschland ca. 40 Mio. männliche Eintagsküken getötet, da bei den Legehybriden lediglich die Produktion von Legehennen im Vordergrund steht und die männlichen Küken demnach keine weitere Verwendung haben.

Die zuvor beschriebene Dissertation von Anne Weissmann schlägt vor, anhand der Östronsulfatkonzentration im embryonalen Harn das entsprechende Geschlecht der sich entwickelnden Küken im jeweiligen Ei zu bestimmen.

Hierzu werden dem Ei, ohne dieses zu beschädigen bzw. zu zerstören, mittels einer 1ml Spritze (Insulinspritze:Omnican 40; B. Braun, Melsungen, Deutschland) ca. 50 µl Allantoisflüssigkeit zwischen dem 7. und 10. Bebrütungstag entnommen und die in der Allantoisflüssigkeit enthaltene Östronsulfatkonzentration bestimmt.

Anhand der so bestimmten Östronsulfatkonzentration kann dann auf das Geschlecht des Kükens im Ei geschlossen werden und das Ei, gegebenenfalls, soweit die Östronsulfatkonzentration auf ein männliches Küken schließen lässt, vor dem am ca. 11. Tag eintretenden Schmerzempfinden des Kükens entsorgt werden.

In der Dissertation hat sich gezeigt, dass sich schon ab dem 9. Bebrütungstag und insbesondere auch am 10. Bebrütungstag in den Eiern signifikant unterschiedliche Östronsulfatkonzentrationen ausbilden, wenn sich in diesen ein weibliches bzw. männliches embryonales Küken befindet.

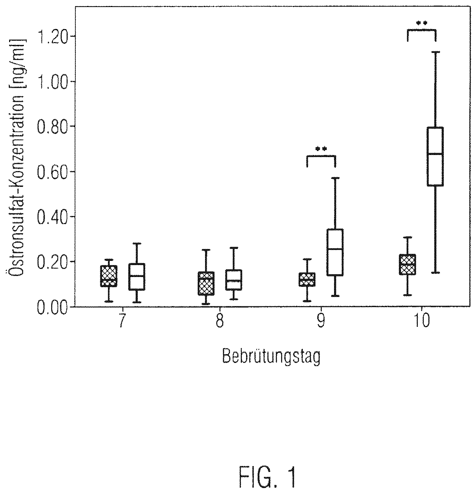

Dieses ist in Fig. 1, welche aus der zuvor beschriebenen Dissertation entnommen ist, dargestellt.

In Fig. 1 zeigen die entsprechenden Balken in den Diagrammen die gemessenen Konzentrationen an Östronsulfat in Eiern mit weiblichen Embryonen (hellgrauer Balken) sowie die Konzentrationen an Östronsulfat in Eiern mit männlichen Embryonen (dunkelgrauer Balken).

So liegt am 9. Tag die durchschnittliche Konzentration an Östronsulfat bei männlichen Embryonen zwischen 0,125 (Medianwert) und bei weiblichen Embryonen bei 0,169 ng/ml.

Am 10. Bebrütungstag liegt die durchschnittliche Konzentration an Östronsulfat bei männlichen Embryonen zwischen 0,193 (Medianwert) und bei weiblichen Embryonen bei 0,667 ng/ml. Die entsprechenden Fehlertoleranzen sind in Figur 1 ebenfalls markiert.

Das in der Dissertation beschriebene Verfahren sei nachfolgend erläutert

Der Nachweis der Östronsulfatkonzentration basiert auf einem sogenannten Enzymimmunassay (ELISA) über die Doppelantikörper-Technik unter Verwendung von 96 well Platten.

Dabei werden die Mikrotiterplatten (96 well Platten) mit 100µl östronsulfatunspezifischem Antikörper (Ziege-Anti-Kaninchen IgG) beschichtet. Diese Flüssigkeit wird über Nacht einer Inkubation unterworfen und anschließend die Mikrotiterplatte gewaschen.

Danach findet die eigentliche Analyse des Mediums (Allantois) statt.

Dieser ELISA beruht auf der Doppelantikörper-Technik; dabei reagiert ein enzymmarkierter Östronsulfat-Antikörperkomplex (Reaktionsprodukt ist Östronsulfat aus dem zu bestimmenden Medium (z.B. Allantois), an welches der östronsulfatspezifische Antikörper und dann das Enzym bindet) mit einem an einer Oberfläche (z.B. Titerplattenboden) gebunden östronsulfatunspezifischen Antikörper.

Dabei handelt es bei dem östronsulfatunspezifischen Antikörper um einen Ziegen-Anti-Kaninchen-IgG.

Die entnommene Allantoisflüssigkeit wird mittels eines Puffers verdünnt und mit einer Enzymlösung und einer Lösung mit einem östronsulfatspezifischer Antikörper (E1S) vermischt.

Als östronsulfatspezifischer Antikörper wird Anti-Östronglucuronid verwendet.

Als Enzym wird Östronglucuronid-Peroxidase verwendet welches sich mit dem östronsulfatspezifischen Antikörper verbindet. Das Östronsulfat in dem zu bestimmenden Medium (z.B. Allantois) verbindet sich ebenfalls mit dem östronsulfatspezifischen Antikörper an welchen das Enzym gebunden ist.

Nach der Reaktion an der Oberfläche (der Mikrotiterplatten) wird diese gespült. So wird gewährleistet, dass lediglich das (enzymmarkierte) Antikörper-gebundene Östronsulfat aus Probe und Standardlösung, weil an die Oberfläche gebunden, zurückbleiben.

Das benutzte Enzym ist in der Lage, eine Farbreaktion zu aktivieren. Über die Intensität der Färbung kann dann die Bestimmung der Östronsulfatkonzentration in der entnommenen Allantoisflüssigkeit mittels Extinktionsmessung im Fotometer durchgeführt werden.

Die Intensität der Färbung steht im Verhältnis zu bekannten Konzentrationen (=Standardkurve).

Die Punkte der Standardkurve liegen zwischen 0,0125 bis 5,0 ng/ml.

Die Enzymlösung, die mit der Probe (z.B. Allantois), welche das Östronsulfat enthält, und dem östronsulfatspezifischen Antikörper (E1S) zusammengegeben wird, weist eine Konzentration von 1:100.000 auf.

Während der 3 stündigen Reaktionszeit wird das Reaktionsprodukt (enzymmarkierter Östronsulfat-Antikörperkomplex) an dem oberflächengebundenen hormon-unspezifischen Antikörper gebunden.

Das zuvor beschriebene Verfahren zur Östronsulfatbestimmung in der Allantoisflüssigkeit funktioniert mittels Doppelantikörper-Technik und die finalen Ergebnisse, inwieweit es sich um ein männlichen oder weiblichen Embryo handelt, liegen nach 4 Stunden vor.

Die Lösung des östronsulfatspezifischen Antikörper (E1S) welche mit der Probe, welche das Östronsulfat enthält und der Enzymlösung zusammengegeben wird, weist eine Konzentration von 1:125.000 auf.

Das zuvor beschriebene Verfahren ist recht komplex und dauert recht lange, bis schlussendlich die entsprechend hohen Östronsulfatkonzentrationen in der entnommenen Allantoisflüssigkeit bestimmt werden kann.

Hierdurch ist das Verfahren für eine großindustrielle Nutzung, bei welcher ein hoher Durchsatz und eine schnelle Analyse von einer großen Menge Eier notwendig sind, routinemäßig eher nicht anwendbar.

Demnach ist es Aufgabe der vorliegenden Erfindung, das zuvor genannte Verfahren dahingehend zu verbessern, dass es auch großtechnisch Anwendung finden kann.

Hierzu schlägt die Erfindung ein Verfahren mit den Merkmalen von Anspruch 1 vor.

Dieses Verfahren bildet das zuvor beschriebene aus der Dissertation bekannte Verfahren dahingehend weiter, dass der östronderivatspezifische Antikörper oberflächengebunden ist. Demnach liegt eine sogenannte Direktbeschichtung mit dem hormonspezifischen Antikörper vor. Somit kann auch die zuvor für den Stand der Technik beschriebene sogenannte Doppelantikörpertechnik nicht zwangsweise verwendet werden. Da der östronderivatspezifische Antikörper nicht zuerst in Lösung mit dem Östronderivat reagiert, sondern schon an eine Oberfläche gebunden vorgelegt wird, kann das Verfahren zur Konzentrationsbestimmung von Östronderivaten bedeutend beschleunigt werden. Bemerkenswerterweise hat sich bei Versuchen gezeigt, dass es möglich ist, den östronderivatspezifischen Antikörper an eine Oberfläche zu binden. Insgesamt kann hierdurch eine Bestimmung der Östronderivatkonzentration innerhalb von max. 2 Stunden durchgeführt werden.

Nachfolgend ist im Allgemeinen die Bestimmung der Östronderivatkonzentration beschrieben. Ein Beispiel eines spezifischen Östronderivates ist das Östronsulfat. Die Bestimmung der Östronsulfatkonzentration ist im speziellen als Beispiel für die Bestimmung der Konzentration eines spezifischen Östronderivates beschrieben. Als Östronderivat kann jedes Östronabbauprodukt oder Östron selber genutzt werden. Anhand der Bestimmung der Östronderivatkonzentration kann eine Unterscheidung von männlichen und weiblichen Embryonen getroffen werden. Zur Anbindung des Östronderivates genügt ein östronderivatspezifischer Antikörper. Sofern als Beispiel auf die Bestimmung der Östronsulfatkonzentration abgestellt wird, kann als Antikörper ein östronspezifischer Antikörper verwendet werden.

Unter spezifischen Antikörpern werden neben den standardmäßigen (nicht modifizierten) spezifischen Antikörpern auch sogenannte spezifische Antikörperkonjugate verstanden. Solche Antikörperkonjugate weisen eine im Vergleich zu den standardmäßigen (nicht modifizierten) spezifischen Antikörpern eine Modifizierung auf, über welche z.B. eine weitere Gruppe (ein Konjugat) angebunden bzw. konjugiert werden kann. Diese spezifischen Antikörperkonjugate sind jedoch immer noch spezifisch für das entsprechende Östronderivat. Demnach wird der Begriff funktional verstanden und schließt eine weitere Modifikation nicht aus. Als Antikörper können mono- oder polyklonale Antikörper eingesetzt werden. Polyklonale Antikörper können in unterschiedlichen Tierarten (Kaninchen, Ziege oder Schaf) gegen das entsprechende Östronsulfat oder deren Derivate gewonnen werden. Für die Erstellung der monoklonalen Antikörper wird die Hybridom-Technik im Kaninchen oder Nagetier genutzt.

Als Konjugat wird vorliegend eine Gruppe verstanden, welche zusätzlich zu dem spezifischen Derivat, auf welches der Antikörper spezifisch ist, an den (modifizierten oder nicht modifizierten) Antikörper bindet. Eine solche Bindung kann z.B. über Brücken mittels Glucuronid angebunden sein, auch andere Linker sind möglich. Dieses Konjugat kann in Abhängigkeit davon, ob an diesen Antikörper auch zusätzlich ein Östronderivat angebunden ist oder nicht, eine unterschiedliche Farbe aufweisen, eine Farbreaktion unterschiedlich katalysieren, bzw. einen Komplex mit unterschiedlicher Farbe bilden. Hierüber kann z.B. die Konzentration des Östronderivates bestimmt werden. Die Farbgebung ist lediglich als Beispiel zu verstehen. Es reicht aus, dass mit Hilfe des Konjugates die Konzentration des Östronderivates bestimmt werden kann. Dies kann auch mittels einer Videoanalyse geschehen, über welche eine Änderung des Zustandes detektiert wird. In Abhängigkeit des an den Antikörper gebunden Östronderivates kann das Konjugat oder der gesamte Komplex aus Konjugat, Antikörper und Östronderivat eine unterschiedliche Aktivität aufweisen. Hierdurch kann die entsprechende Konzentration an Östronderivat bestimmt werden.

Gemäß der Erfindung liegt der östronderivatspezifische Antikörper direkt an einer Oberfläche gebunden vor. Der östronderivatspezifische Antikörper wird unmittelbar an eine Oberfläche angebunden. Somit ist, anders als im Stand der Technik, kein unspezifischer Antikörper zwischen der Oberfläche und dem spezifischem Antikörper vorgesehen.

Gemäß einer vorteilhaften Weiterbildung kann das Konjugat einen kolloidalen Marker enthalten. Ein solcher kolloidaler Marker kann kolloidales Gold, kolloidaler Latex oder ein anderer kolloidaler Stoff sein. Dieser kolloidale Marker kann das Konjugat selber bilden oder auch nur ein Teil des Konjugates sein. Soweit Gold als kolloidaler Marker verwendet wird, wird dieses als Immunogold-Konjugation bezeichnet. Aufgrund der intensiven Farbigkeit des kolloiden Goldes auf der einen Seite und der spezifischen Bindungseigenschaften der Antikörper auf der anderen Seite lassen sich Antikörper-Gold-Konjugate für die Bestimmung der Östronderivatkonzentration gut einsetzen. Gold-Konjugate (der Antikörper an den unmittelbar oder mittelbar kolloides Gold konjugiert ist) weisen eine gute Stabilität auf, da die Bindung zwischen Protein und Goldteilchen relativ stark ist. Somit wird eine höhere Sensitivität erreicht. Es können auch Latex-Konjugate, die Latexkolloide enthalten oder ein Latexkolloid sind, genutzt werden, da sich das Label quasi maßschneidern lässt. Latex-Partikel können in beliebigen Farben hergestellt werden.

Gemäß einer vorteilhaften Weiterbildung nach Anspruch 3 kann das Konjugat ausgewählt aus der folgenden Gruppe von Verbindungen sein: goldmarkierte Steroid-Protein-Verbindung, kolloidales Gold, latexmarkiertes Steroid-Protein-Verbindung, kolloidaler Latex. Steroid-Protein-Verbindungen sind z.B. Enzyme bzw. modifizierte Enzyme. Die Enzyme sind z.B. dahingehend chemisch modifiziert, dass der kolloidale Marker besser an diese anbinden kann. Jegliche andere Modifikation ist aber auch denkbar. Beispiele von Steroid-Protein-Verbindungen sind: Östron-glucuronid Peroxidase, Östron-carboxymethyl-oxim, Östron-carboxymethyl-ether. Dieses Konjugat kann dann an den Antikörper angebunden werden. Diese Konjugate liegen z.B. in Lösung vor. Auch kolloide Mischungen sind in diesem Zusammenhand als Lösungen bezeichnet.

Gemäß der Erfindung ist die mit dem östronderivatspezifischen Antikörper zu beschichtende Oberfläche eine Glas, Glasfiber-, Papier- oder Polypropylenoberfläche.

Gemäß einer vorteilhaften Weiterbildung kann der östronderivatspezifische Antikörper ein östronsulfatspezifischer Antikörper sein, um damit die Östronsulfatkonzentration der entnommenen Allantoisflüssigkeit bestimmt werden. Wie zuvor bereits beschrieben, ist diese Variante eine Untergruppe des östronderivatspezifischen Antikörpers. Sowie nachfolgend auf den östronsulfatspezifischen Antikörper abgestellt wird, gilt dies auch im Allgemeinen für den östronderivatpezifischen Antikörper (in modifizierter oder nicht modifizierter Form zur Konjugation mit dem Konjugat) und/oder auch nicht nur für Östronsulfat sondern für jegliche Östronderivate.

Gemäß einer vorteilhaften Weiterbildung kann die Konjugatlösung eine Enzymlösung sein, mit einem Enzym, welches in der Lage ist einen hinzugefügten Farbstoff zu aktivieren und, dass die Bestimmung der Östronsulfatkonzentration der entnommenen Allantoisflüssigkeit mittels fotometrischer Bestimmung der Konzentration des mittels des Enzyms aktivierten Farbstoffes durchgeführt wird.

Wie zuvor bereits beschrieben, ist diese Variante bei der als Konjugat ein Enzym verwendet wird eine Untergruppe der allgemeinen Form des Konjugates. An das Enzym kann auch ein kolloidaler Marker angebunden sein. Soweit weiter unten in Bezug auf die Unteransprüche auf "Enzym" abgestellt wird, kann auch jegliches andere Konjugat verwendet werden. Die Konzentrationen bzw. Vorteile gelten auch in der allgemeinen Form für die verwendeten Konjugate.

Auch der östronsulfatspezifische Antikörper ist oberflächengebunden. Demnach liegt eine sogenannte Direktbeschichtung mit dem hormonspezifischen Antikörper vor. Somit ist es nicht notwendig die zuvor für den Stand der Technik beschriebene sogenannte Doppelantikörper-technik zu verwenden. Dadurch, dass der östronsulfatspezifische Antikörper nicht zuerst in Lösung mit dem Östronsulfat reagiert, sondern schon an eine Oberfläche gebunden vorgelegt wird, kann das Verfahren zur Konzentrationsbestimmung von Östronsulfat bedeutend beschleunigt werden. Bemerkenswerterweise hat sich bei Versuchen gezeigt, dass es möglich ist, den östronsulfatspezifischen Antikörper an eine Oberfläche zu binden. Insgesamt kann hierdurch eine Bestimmung der Östronsulfatkonzentration innerhalb von max. 2 Stunden durchgeführt werden.

Nachfolgend wird in Bezug auf die Weiterbildungen die Variante beschrieben, in der das Östronderivat Östronsulfat ist, und entsprechend der Antikörper ein östronsulfatspezifischer Antikörper ist; wobei als Konjugat ein Enzym verwendet wird, welches in der Lage ist, einen hinzugefügten Farbstoff zu aktivieren und, dass die Bestimmung der Östronsulfatkonzentration der entnommenen Allantoisflüssigkeit mittels fotometrischer Bestimmung der Konzentration des mittels des Enzyms aktivierten Farbstoffes durchgeführt wird.

Gemäß einer vorteilhaften Weiterbildung kann die Allantoisflüssigkeitsprobelösung und die Enzymlösung, welche eine Verdünnung von zwischen 1:60.000 und 1:90.000 aufweist, zu dem oberflächengebunden östronsulfatspezifischen Antikörper gegeben werden.

Durch die im Vergleich zum vorgenannten Stand der Technik (Doppelantikörper-Technik) geringere Verdünnung (höhere Konzentration) der Enzymlösung bei der Direktbeschichtung hat sich gezeigt, dass wenn die Probelösung (Allantoisflüssigkeit), und die Enzymlösung mit der mit dem östronsulfatspezifischen Antikörper beschichteten Oberfläche in Kontakt gebracht werden, eine schnellere und somit effektivere Reaktion zwischen Enzym bzw. Östronsulfat aus der Probe und dem hormonspezifischen Antikörper stattfindet.

Als östronsulfatspezifischer Antikörper wird vorzugsweise Anti-Östronglucuronid verwendet. Für die Immunisierung (Antikörperherstellung) wird vorzugsweise ein Trägermolekül BSA (Rinderalbulmin) mittels Glucuronid an das Östron gekoppelt. Demnach wird der östronsulfatspezifische Antikörper vorzugsweise gegen Östronglucuronid hergestellt.

Als Enzym wird vorzugsweise Östronglucuronid Peroxidase verwendet. Bevorzugte Bereiche der Verdünnung der Enzymlösung können zwischen 1:70.000 und 1:80.000, insbesondere bei ca. 1:75.000 +/- 1.000 liegen. Soweit auf einer Verdünnung der Enzymlösung abgestellt wird, kann diese Verdünnung mit Hilfe von Wasser (vorzugsweise demineralisiert) oder einer Pufferlösung geschehen.

Gemäß einer nicht erfindungsgemäßen Weiterbildung kann 2 µl bis 50 µl

Allantoisflüssigkeit aus dem Ei entnommen werden. Ein weiterer nicht erfindungsgemäßer Mengenbereich ist 5 µl bis 20 µl.

Es hat sich verwunderlicherweise gezeigt, dass, auch bei einer im Vergleich zum Stand der Technik geringeren Entnahmemenge, noch zuverlässig die unterschiedlichen Östronsulfatkonzentrationen bestimmt werden können.

Je weniger Allantoisflüssigkeit (z.B. < 50 µl) entnommen wird, eine desto geringere oder gar keine Verschlechterung in der Schlupfrate konnten festgestellt werden, im Vergleich zu Eiern, die nicht beprobt wurden.

Gemäß einer vorteilhaften Weiterbildung nach Anspruch 10 kann die aus dem Ei entnommene Allantoisflüssigkeit unverdünnt mit dem östronsulfatspezifischen Antikörper und dem Enzym zusammengebracht werden.

Die Proben (Allantois) werden bei der Direktbeschichtungsmethode entsprechend der Erfindung unverdünnt eingesetzt. Somit kann die Allantoisflüssigkeit, im Wesentlichen so wie sie aus dem Ei entnommen ist, eingesetzt werden. Im Wesentlichen so wie sie ist, schließt eine mechanische Filterung nicht aus. Bevorzugt jedoch wird auch eine solche Filterung unterlassen, da sich bemerkenswerterweise herausgestellt hat, dass auch eine ungefilterte und unverdünnte Probe die spätere Reaktion nicht nachteilig beeinflusst.

Diese unverdünnte Allantoisflüssigkeit kann dann zusammen mit dem Enzym auf die beschichtete Oberfläche gebracht werden.

Gemäß einer nicht erfindungsgemäßen Weiterbildung kann zwischen 2 µl und 50 µl Probelösung und zwischen 30 µl und 70 µl Enzymlösung zusammengegeben werden. Weitere Werte sind zwischen 5 µl und 20 µl, insbesondere 50 µl. Vorzugsweise werden gleiche Mengen an Probelösung und Enzymlösung vermischt und mit der beschichteten Oberfläche in Kontakt gebracht.

Gemäß einer vorteilhaften Weiterbildung nach Anspruch 12 kann eine Lösung des östronsulfatspezifischen Antikörpers in einer Verdünnung von 1:90.000 bis 1:150.000 auf die zu beschichtende Oberfläche gegeben wird.

Bevorzugte Bereiche der Verdünnung des östronsulfatspezifischen Antikörpers kann zwischen 1:100.000 und 1:130.000, insbesondere bei ca. 1:125.000 +/- 1.000 liegen.

Damit der östronsulfatspezifische Antikörper auf der Oberfläche immobilisiert wird, sollte die Konzentration, im Vergleich zum Stand der Technik deutlich höher sein, als wenn die Antikörper-Lösung lediglich für eine In-Situ Reaktion verwendet wird.

Gemäß einer vorteilhaften Weiterbildung kann zum Beschichten der Oberfläche mit dem östronsulfatspezifischen Antikörper, die Lösung des östronsulfatspezifischem Antikörpers für 4 bis 14 Stunden auf die zu beschichtende Oberfläche einwirken. Es hat sich gezeigt, dass die besten Ergebnisse der Immobilisierung erreicht werden können, wenn die Lösung des östronsulfatspezifischen Antikörpers 8 bis 13 Stunden auf die Oberfläche einwirkt.

Im Anschluss an diese Beschichtung kann vorzugsweise eine Nachbeschichtung mit 0,1% BSA-Lösung (Bovines Serum Albumin) durchgeführt werden. Dies kann wird insbesondere wenn es sich bei der zu beschichtenden Oberfläche um Mikrotiterplatten handelt wie folgt durchgeführt werden: 280 µl +/- 50 µl BSA Lösung (Fraktion V, Firma Serva) werden pro Kavität für ca. 30 Minuten inkubiert, danach entleert.

Gemäß einer vorteilhaften Weiterbildung kann nach Einwirkung der Lösung des östronsulfatspezifischen Antikörpers und gegebenenfalls das BSA, die beschichtete Oberfläche lediglich einmal mit Polysorbat 80 Lösung gewaschen wird. Soweit es sich bei der zu beschichtenden Oberfläche um die Kavität einer Mikrotitierplatte handelt, reichen einmaliges Spülen mit ca. 300 µl pro Kavität der Mikrotiterplatte aus. Eine Spülung kann mit einer Menge von 50 µl bis 300 µl durchgeführt werden.

Polysorbat 80 ist eine polyoxyethylierte Verbindung, die sich von Sorbitol und Ölsäure ableitet. Polysorbat 80 ist auch unter dem Namen TWEEN 80 bekannt.

Es hat sich gezeigt, das nach einem einzigen mal Waschen im Wesentlichen aller nicht immobilisierte östronsulfatspezifische Antikörpers entfernt werden konnte und auch noch genug östronsulfatspezifische Antikörper in immobilisierter Form vorliegt und nicht ausgewaschen wurde.

Gemäß der Erfindung kann die zu beschichtende Oberfläche eine Kavität einer Mikrotestplatte oder Vlies sein.

Solche Mikrotestplatten sind allgemein bekannt und weisen eine Vielzahl von Kavitäten (auch wells genannt auf, in denen die Probelösung und die Enzymlösung vermischt werden können.

Diese Kavitäten dienen als Mikroreaktoren bei der Bestimmung. Es können vorzugsweise 96-well, 384-well Mikrotestplatten (Firma nunc) verwendet werden.

Alternativ anstelle der Verwendung solcher Kavitäten kann auch ein Vlies auf Verwendung finden, welcher dann in einer Testkassette eingebaut sein kann.

Bei einer solchen Verfahrensführung handelt es sich um eine Abwandlung der als Lateral-Flow Technik bekannten Technik.

Gemäß der Erfindung kann die Probelösung und die Enzymlösung in die beschichtete Kavität der Mikrotestplatte gegeben werden und das Reaktionsgemisch nach der Reaktion ausgegossen werden.

Vorzugsweise sind die mit östronsulfatspezifischem Antikörper beschichteten Kavitäten direkt mit der entsprechenden Mengen an Probelösung und Enzymlösung versetzt.

Es hat sich gezeigt, dass die Mikrotestplatten mit den beschichteten Kavitäten sogar bei unterhalb von -17°C insbesondere bei ca. -17 bis -23°C über Monate gelagert werden können, ohne sich ungünstig im Hinblick auf die spätere Reaktion mit dem Östronsulfat zu verändern.

Somit ist es erstmals möglich die Mikrotestplatten mit den beschichteten Kavitäten schon lange vor der Bestimmung vorzubereiten.

Gemäß einer vorteilhaften Weiterbildung kann nach Reaktion zwischen östronsulfatspezifischem Antikörper, Östronsulfat in der Probe und Enzym, das generierte immobilisierte Reaktionsprodukt lediglich zweimal mit Polysorbat 80 gewaschen werden.

Es hat sich gezeigt, dass ein zweimaliges Waschen ausreicht, um alle ungewünschten nicht an den östronsulfatspezifischem Antikörper gebundenen Moleküle zu entfernen, so dass das angebundene Östronsulfat im Hinblick auf dessen Konzentration sehr genau bestimmt werden kann. Soweit es sich bei der zu beschichtenden Oberfläche um die Kavität einer Mikrotitierplatte handelt, reichen zweimaliges Spülen mit zu 300 µl, insbesondere 300 µlpro Kavität der Mikrotiterplatte aus.

Gemäß einer vorteilhaften Weiterbildung kann das gewaschene immobilisierte Reaktionsprodukt mit einer Lösung, welche einen mittels des Enzym aktivierbaren Farbstoff enthält, zusammengebracht werden und die Farbstoffkonzentration nach einer vorgegebenen Zeit fotometrisch bestimmt werden.

Ein solcher Farbstoff, der mittels des angebundenen Enzyms aktiviert werden kann, kann wie folgt hergestellt werden. Eine Substanz A aus H₂O₂-Hamstoff/Na₂-HPO₄/Citronensäure wird mit einer Substanz B aus Tetramethylbenzidin/DMSO/Citronensäure, je in gleichen Teilen, zusammengemischt und beschichtete Oberfläche, insbesondere die Kavität gegeben. Das Enzym katalysiert eine Farbreaktion, über welche auf die Enzymkonzentration und somit mittelbar auf die Östronsulfatkonzentration geschlossen werden kann.

Mittels fotometrischer Methoden kann die relative Farbstoffkonzentration mit Bezug auf die Standardpunkte (0,125 - 1,0 ng/ml) bestimmt werden.

Es werden Standardpunkte (Vergleichsprobelösungen) mit bekannten Konzentrationen an Östronsulfat (0,125 - 1,0 ng/ml) hergestellt, welche auf dieselbe Weise, wie die entnommene Allantoisflüssigkeit mit dem Enzym und dem immobilisierten östronsulfatspezifischem Antikörper reagieren. Es wird eine Kalibrationskurve aus den gemessenen fotometrischen Daten (Absorption) erstellt. Die bei der zu untersuchenden Probe gemessenen fotometrischen Daten (Absorption), werden mit der Kalibrationskurve verglichen, um so die Konzentration des Östronsulfates zu bestimmen.

Die fotometrische Bestimmung läuft vorzugsweise wie folgt ab:
Das Enzym (z.B. Östronglucuronid-Peroxidase) aus der Enzymlösung bindet an den oberflächengebundenen Antikörper.
Das antikörpergebundene Enzym ergibt nach der Farbreaktion den höchsten Extinktions-Messwert im Fotometer, wenn kein Östronsulfat (aus Eichreihe oder Allantoisprobe) an den Antikörper gebunden ist.
Mit steigender Östronsulfatkonzentration bindet weniger Enzym an den oberflächengebundenen Antikörper, wodurch bei der späteren Farbreaktion die Extinktion abnimmt.
Es kann hieraus bevorzugt mittels Computerauswertung die Östronsulfat-Konzentration ermittelt werden kann.
Vorliegen ist demnach die Färbung (gemessene Extinktion) umgekehrt proportional zur Eichkurve.

Gemäß einer vorteilhaften Weiterbildung kann die fotometrische Messung der Farbreaktion zur Bestimmung der die Farbstoffkonzentration in der Kavität der Mikrotestplatte durchgeführt werden.

Hierdurch kann das Verfahren weiter beschleunigt werden.

Gemäß einer vorteilhaften Weiterbildung kann die Mikrotestplatte eine Vielzahl von Kavitäten enthalten, und die entnommene Allantoisflüssigkeit von unterschiedlichen Eiern in unterschiedliche Kavitäten gegeben werden, und auf automatisierte Weise die Östronsulfatkonzentration in den verschiedenen Eiern bestimmt werden, indem die die fotometrische Messung in den unterschiedlichen Kavitäten automatisiert erfolgt und/oder der Schritt des Zusammengebens von Enzym, Probenlösung in die beschichtete Kavität automatisiert erfolgt.

Hierdurch kann das Verfahren weiter beschleunigt werden.

In den weiteren Weiterbildungen wird die allgemeine Variante des Verfahrens beschrieben, welche bereits näher erläutert wurde. Die entsprechenden Konzentrationen und die Verfahrensführung kann im Vergleich zu der speziellen Variante leicht unterschiedlich sein. Diese Variante ist nicht auf eine fotometrische Bestimmung beschränkt, sondern es kann jedes beliebige Verfahren verwendet werden, mit dem mit Hilfe des Konjugats auf die Konzentration des Östronderivates geschlossen werden kann. Eine Methode in welcher eine Videokamera zur Bestimmung der konzentrationsabhängigen Eigenschaften verwendet wird ist ebenso denkbar.

Vorteilhafte Weiterbildungen der Erfindung ergeben sich aus den nachfolgend erörterten Beispielen in Verbindung mit der Zeichnung.

In dieser zeigen
Fig. 1 aus dem Stand der Technik bekannte gemessene Konzentrationen an Östronsulfat in Eiern mit weiblichen Embryonen sowie die Konzentrationen an Östronsulfat in Eiern mit männlichen Embryonen,
Fig. 2 eine schematische Abbildung einer Embryonalhülle eines Huhnes am 10. bis 11. Bebrütungstag,
Fig. 3 die Entnahme von Allantoisflüssigkeit mittels einer Insulinspritze, und
Fig. 4 eine Eichkurve zur Bestimmung des Östronsulfatgehaltes gemäß der vorliegenden Erfindung.

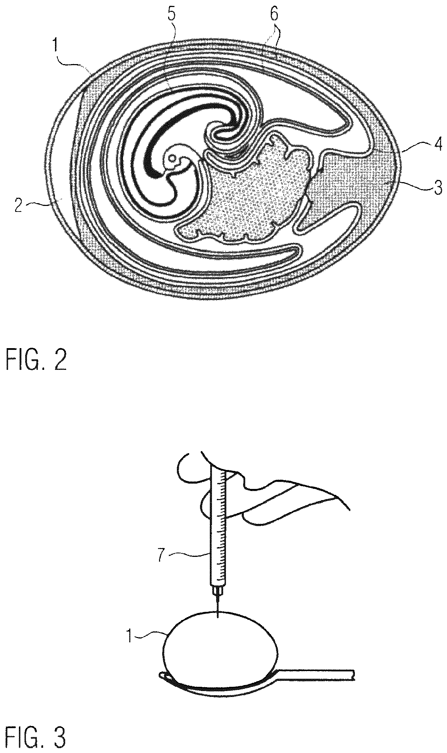

Figur 2 zeigt eine Embryonalhülle eines Huhnes am 10. Bis 11. Bebrütungstag.

Das in seiner Querschnittsansicht schematisch dargestellte Ei weist eine Kalkschale 1 mit einer Schalenhaut auf, innerhalb der Kalkschale 1 ist eine Luftkammer 2 und Eiweiß 3 eingeschlossen.

Im Eiweiß ist das sogenannte Chorion 4 gelagert, in welchem das embryonale Küken 5 und die Allantois 6 beherbergt ist.

Die Stoffwechselprodukte des Embryos werden hauptsächlich in Form von Ammoniumharnstoff und Harnsteinsäure ausgeschieden. Ein weiteres Abbauproduktprodukt ist das zuvor beschrieben Östronsulfat. Auch wenn im Stand der Technik die Konzentration von Östronsulfat bestimmt wird, kann jedes beliebige Östronabbauprodukt oder Östron selber genutzt werden um eine Unterscheidung von männlichen und weiblichen Embryonen zu treffen. Deshalb ist in Anspruch 1 lediglich ein östronderivatspezifischer Antikörper definiert. Deshalb wird das erfindungsgemäße Verfahren auch im Allgemeinen für Östronderivate beschrieben.

Nachfolgend ist als spezielles Beispiel lediglich die Variante beschrieben, in der das Östronderivat Östronsulfat ist, und entsprechend der Antikörper ein östronsulfatspezifischer Antikörper ist; wobei als Konjugat ein Enzym verwendet wird, welches in der Lage ist, einen hinzugefügten Farbstoff zu aktivieren und, dass die Bestimmung der Östronsulfatkonzentration der entnommenen Allantoisflüssigkeit mittels fotometrischer Bestimmung der Konzentration des mittels des Enzyms aktivierten Farbstoffes durchgeführt wird.

Diese Beschreibung gilt jedoch auch für jedes Östronderivat, bzw. jedes Konjugat und es kann jedes Verfahren zur Bestimmung der Konzentration an Östronderivat Verwendung finden. Eine fotometrische Messung ist nicht essentiell.

In Fig. 3 ist ein Ei dargestellt, in welches eine Insulinspritze 7 eingestochen ist in einer derartigen Position, dass aus der Allantois entsprechende Flüssigkeit entnommen werden kann.

Nachfolgen werden Beispiele von einzelnen Komponenten beschrieben.

### Herstellung der Probelösung

In einem Beispiel, welches nicht Teil der Erfindung ist, werden mittels einer Spitze 2 µl bis 50 µl, oder 5 µl bis 20 µ

Allantoisflüssigkeitsprobe aus dem Ei entnommen.

Die entnommene Allantoisflüssigkeit wird unverdünnt eingesetzt und bildet so die Probelösung, welche mit der Enzymlösung auf die beschichtete Oberfläche gebracht wird.

### Herstellung der Enzymlösung

Als Enzym wurde vorliegend Östronglucuronid Peroxidase verwendet.

Das Östronsulfat in der Probe verbindet sich mit dem östronsulfatspezifischer Antikörper an welchen auch das Enzym bindet.

Bevorzugte Bereiche der Verdünnung der Enzymlösung können zwischen 1:60.000 und 1:90.000, bevorzugt zwischen 1:70.000 und 1:80.000, insbesondere bei ca. 1:75.000 +/- 1.000 liegen.

Das Enzym wurde mit Puffer verdünnt.

Die Pufferlösung enthielt 7,12 g Natriumhydrogenphosphat (Merck Deutschland), 8,5 g Natriumchlorid (Merck, Deutschland), 1,0 g Rinderserumalbumin (Serva, Deutschland) und 1000 ml Wasser. Die Pufferlösung wies einen pH-Wert von 7,2 auf.

### Oberflächenbeschichtung / Lagerung

Eine Lösung des östronsulfatspezifischen Antikörpers kann bei einer Verdünnung von 1:90.000 bis 1:150.000, vorzugsweise zwischen 1:100.000 und 1:130.000, insbesondere bei ca. 1:125.000 +/- 1.000 liegen wurde auf die zu beschichtende Oberfläche gegeben wird.

Als östronsulfatspezifischer Antikörper kann vorzugsweise Anti-Östronglucorunid verwendet werden, sprich der Antikörper ist gegen Östronglucuronid(-BSA) gerichtet und wurde im Kaninchen gewonnen.

Die zu Beschichtende Oberfläche war eine Kavität einer Mikrotestplatte. Es können vorzugsweise 96-well, 384-well, Mikrotestplatten (Firma nunc) verwendet werden.

Alternativ zu den Mikrotestplatten kann auch ein Vlies verwendet werden, an den der östronsulfatspezifischer Antikörper angebunden ist, und die Lösungen können auf den Vlies in Kassetten gegeben werden. Dabei handelt es sich um eine Abwandlung der als Lateral-Flow-Assay bekannten Technik, die ein kompetetives Assay-System darstellt. Es können als zu beschichtende Oberfläche eine Glas, Glasfiber-, Papier- oder Polypropylenoberfläche verwendet werden.

In dem Fall werden die Lösungen auf Glas, Glasfiber-, Papier- Polypropylen-, bzw. Vliesoberflächen aufgebracht, um die Reaktion zu beginnen. Bei diesem System ist ein Spülvorgang, wie im Folgenden beschrieben nicht notwendig. Nach Zugabe der Markerlösung erfolgt ein Farbumschlag, der dokumentiert wird.

Zwischen 50 µl und 150 µl, insbesondere 100 µl Lösung des östronsulfatspezifischen Antikörpers wurden in jede Kavität gegeben und über Nacht bei Raumtemperatur stehen gelassen. Hiernach wurden die Kavitäten der Mikrotestplatten ausgeleert mit zwischen 100 µl und 500 µl, insbesondere 300 µl Assaypuffer für 30 Minuten bei 150/min geschüttelt.

Danach wurde der Assaypuffer aus der Mikrotiterplatte ausgeleert.

Die Kavitäten der Platten sind nun mit dem östronsulfatspezifischen Antikörpers beschichtet. Diese Platten können ohne Schaden bei mindestens -17°C insbesondere bei ca. -17 bis -23°C über Monate gelagert werden bevor sie Verwendung finden.

### Reaktion

Die Probelösung und die Enzymlösung wurden in die beschichteten Kavitäten gegeben und für 75 Minuten unter schütteln inkubiert. Nach dieser Zeit hatte alles stöchiometrisch reagiert und das Östronsulfat war an den östronsulfatspezifischen Antikörper auf der Oberfläche gebunden. Es wurden jeweils gleiche Mengen an zwischen 2 µl und 50 µl Probelösung und zwischen 30 µl und 70 µl Enzymlösung zusammengegeben. Erfindungsgemäße Werte sind zwischen 5 µl und 15 µl.

Nach der Reaktion wurde die Mikrotestplatte ausgeleert und 2-mal mit TWEEN 80 Lösung gewaschen.

### Verfahren zur Konzentrationsbestimmung von Östronsulfat

Das gewaschene immobilisierte Reaktionsprodukt wurde mit einer Lösung, welche einen mittels des Enzyms aktivierbaren Farbstoff enthält, zusammengebracht und die Farbstoffkonzentration nach einer vorgegebenen Zeit fotometrisch bestimmt.

Ein solcher Farbstoff, der mittels des angebundenen Enzyms aktiviert werden kann, kann wie folgt hergestellt werden.

Eine Substanz A aus H₂O₂-Harnstoff/Na₂-HPO₄/Citronensäure wird mit einer Substanz B aus Tetramethylbenzidin/DMSO/Citronensäure je in gleichen Teilen zusammengemischt und auf die gespülte Oberfläche gegeben. Das Enzym katalysiert eine Farbreaktion, über welche auf die Hormonkonzentration und somit mittelbar auf die Östronsulfatkonzentration geschlossen werden kann.

Mittels fotometrischer Methoden kann die relative Farbstoffkonzentration bestimmt werden.

Es werden Standardlösungen mit bekannten Konzentrationen an Östronsulfat hergestellt, welche auf dieselbe Weise wie die entnommene Allantoisflüssigkeit mit dem Enzym und dem immobilisierten östronsulfatspezifischem Antikörper reagieren. Es wird eine Kalibrationskurve aus den gemessenen fotometrischen Daten (Absorption) erstellt. Die bei der zu untersuchenden Probe gemessenen fotometrischen Daten (Absorption), werden mit der Standardkurve verglichen um so die Konzentration des Östronsulfates zu bestimmen.

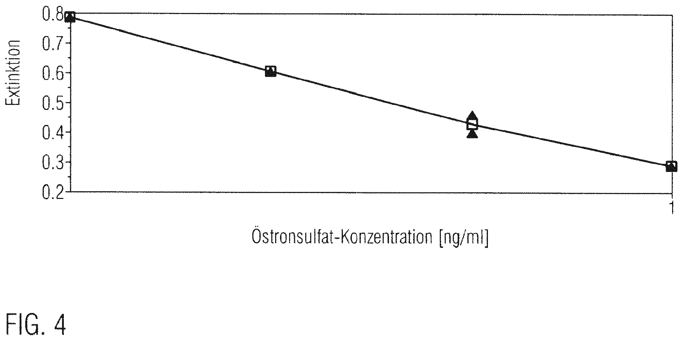

Zur Bestimmung der Östronsulfatkonzentration wurden Eichproben von bekannten Mengen Östronsulfat hergestellt und auf dieselbe Weise wie zuvor beschrieben behandelt. Aus den für diese Eichroben gemessenen Daten wurde die in Figur 4, abgebildete Eichkurve erhalten.

Durch Vergleich der Ergebnisse der fotometrischen Messung der Proben mit unbekanntem Östronsulfatgehalt mit der Eichkurve kann auf die Östronsulfatkonzentration in der Probe geschlossen werden.

Als Spektrometer wurde ein sogenannter Viktor 21420 Multilabelcounter verwendet, die gemessenen Werte wurden mit der Software "Workout" (Perkin Elmer) bestimmt.

### Östronsulfatkonzentration zur Bestimmung des Geschlechts

Mit dem zuvor beschriebenen Verfahren können Unterschiede in der Östronsulfatkonzentration der Allantiolisflüssigkeit von 0,1 ng/ml unterschieden werden, welches mit dem bisher bekannten Verfahren nicht möglich ist.

Demnach kann eine genauere Bestimmung zwischen weiblichen und männlichen Embryonen durchgeführt werden.

Zur Verdeutlichung ist dieser Sachverhalt in nachfolgend abgebildeten Tabellen 1 und 2 dargestellt. Hierbei zeigt Tabelle 1, die Konzentrationen von Östronsulfat bzw. Östrandiol, in der zu verschiedenen Bebrütungsdauern entnommenen Alantoisflüssigkeitsproben.

**Tabelle 1: Konzentrationen von Östronsulfat bzw. Östrandiol in zu verschiedenen Bebrütungsdauern entnommenen Alantoisflüssigkeitsproben.**

| **Beprobungstag/ Embryonaltag** | **Geschlecht** | **Östronsulfat (ng/ml)** | | | | **Östradiol (pg/ml)** | | | |
|---|---|---|---|---|---|---|---|---|---|
| | | median | P 25 | P75 | p | median | P25 | P75 | p |
| 7 | Männlich | 0,11 | 0,07 | 0,14 | 0,91 | - | - | - | - |
| | Weiblich | 0,12 | 0,53 | 0,14 | | - | - | - | |
| 8 | Männlich | 0,12 | 0,10 | 0,19 | 0,29 | 40,95 | 28,38 | 50,75 | 0,56 |
| | Weiblich | 0,15 | 0,11 | 0,23 | | 32,40 | 29,95 | 38,90 | |
| 9 | Männlich | 0,14 | 0,09 | 0,17 | < 0,01 | 157,65 | 54,35 | 326,75 | 0,01 |
| | Weiblich | 0,21 | 0,14 | 0,35 | | 329,50 | 215,18 | 370,25 | |
| 10 | Männlich | 0,17 | 0,14 | 0,22 | < 0,01 | 101,75 | 43,68 | 116,10 | < 0,01 |
| | Weiblich | 0,68 | 0,57 | 0,81 | | 158,90 | 125,85 | 214,85 | |

In obiger Tabelle 1 zeigen median, P25 sowie P75 die die jeweiligen sogenannten Quantile zwischen P25 und P75 liegen 50% der Werte der Gemessenen Verteilung.

Beispielsweise beträgt der Medianwert der Östronsulfatkonzentration bei männlichen Embryonen am 7. Bebrütungstag 0,11 ng Östronsulfat pro ml entnommener Probe. Der Medianwert der Östronsulfatkonzentration bei weiblichen Embryonen am 7. Bebrütungstag beträgt 0,12 ng Östrobnsulfat pro ml entnommener Probe.

Beispielsweise beträgt der Medianwert der Östronsulfatkonzentration bei männlichen Embryonen am 9. Bebrütungstag 0,14 ng Östronsulfat pro ml entnommener Probe. Der Medianwert der Östronsulfatkonzentration bei weiblichen Embryonen am 8. Bebrütungstag beträgt 0,21 ng Östrobnsulfat pro ml entnommener Probe.

Entsprechende Konzentrationen sind auch für Östrandiol in der Tabelle angegeben.

In Tabelle 2 sind die Grenzwerte der Östronsulfatkonzentzrationen dargestellt, die ein männlichen Embryo bzw. einen weiblichen Embryo anzeigen.

**Tabelle 2: Grenzwerte der Östronsulfatkonzentzrationen, die ein männlichen Embryo bzw. einen weiblichen Embryo anzeigen.**

| **Beprobungstag/ Embryonaltag** | **Geschlecht** | **Östronsulfat (ng/ml) GW (Grenzwerte)** |
|---|---|---|
| 7 | Männlich | Kein Unterschied |
| | Weiblich | |
| 8 | Männlich | ≤ 0,10 |
| | Weiblich | > 0,10 |
| 9 | Männlich | ≤ 0,17 |
| | Weiblich | > 0,17 |
| 10 | Männlich | ≤ 0,26 |
| | Weiblich | > 0,26 |

In obiger Tabelle 2 sind die Unterschiede in der Östronsulfatkonzentration in Abhängigkeit der Bebrütungstage angegeben.

Am 7. Bebrütungstag kann mit dem erfindungsgemäßen Verfahren noch kein Unterschied festgestellt werden.

Am 8. Bebrütungstag beträgt der Grenzwert für männliche Embyonen 0,10 ng/ml oder weniger Östronsulfat in der Anantoisflüssigkeit. Bei einer Konzentration von mehr als 0,10 ng/ml Östronsulfat handelt es sich um einen weiblichen Embryo.

Mittels des erfindungsgemäßen Verfahrens kann noch früher ein Unterschied in der Östronsulfatkonzentration detektiert werden sowie zudem die Bestimmung schneller durchgeführt werden.

**Bezugszeichenliste:**

| | |
|---|---|
| Kalkschale | 1 |
| Luftkammer | 2 |
| Eiweiß | 3 |
| Chorion | 4 |
| embryonale Küken | 5 |
| Allantois | 6 |
| Insulinspritze | 7 |

## Patentansprüche

1. Verfahren zur *in ovo* Geschlechterbestimmung von Küken, enthaltend die Schritte:
Entnahme einer Probelösung von Allantoisflüssigkeit aus einem Ei;
Zusammenbringen der aus dem Ei entnommenen Probelösung mit einem östronderivatspezifischen Antikörper und einer Konjugatlösung mit einem an den Antikörper bindenden Konjugat; und
Bestimmung der Östronderivatkonzentration der entnommenen Allantoisflüssigkeit mit Hilfe des an den Antikörper gebundenen Konjugats;
**dadurch gekennzeichnet, dass**
der östronderivatspezifische Antikörper direkt an einer Oberfläche gebunden vorliegt, wobei die mit dem östronderivatspezifischen Antikörper zu beschichtende Oberfläche eine Vlies-, Glas-, Glasfiber-, Papier-, oder Polypropylenoberfläche oder eine Kavität einer Mikrotestplatte ist, wobei zwischen 5 µl und 15 µl Probelösung aus dem Ei entnommen werden und
die Probelösung unverdünnt mit dem östronderivatspezifischen Antikörper und
der Enzymlösung zusammengebracht wird.

2. Verfahren nach Anspruch 1, **dadurch gekennzeichnet, dass** das Konjugat einen kolloidalen Marker enthält; und/oder dass das Konjugat ausgewählt ist aus der folgenden Gruppe von Verbindungen: goldmarkierte Steroid-Protein-Verbindung, kolloidales Gold, latexmarkierte Steroid-Protein-Verbindung, kolloidaler Latex.

3. Verfahren nach einem der vorherigen Ansprüche, **dadurch gekennzeichnet, dass** der östronderivatspezifische Antikörper ein östronsulfatspezifischer Antikörper ist und die Östronsulfatkonzentration der entnommenen Allantoisflüssigkeit bestimmt wird.

4. Verfahren nach Anspruch 3, **dadurch gekennzeichnet, dass** die Konjugatlösung eine Enzymlösung ist, mit einem Enzym, welches in der Lage ist, einen hinzugefügten Farbstoff zu aktivieren, und dass die Bestimmung der Östronsulfatkonzentration der entnommenen Allantoisflüssigkeit mittels fotometrischer Bestimmung der Konzentration des mittels des Enzyms aktivierten Farbstoffes durchgeführt wird.

5. Verfahren nach Anspruch 4, **dadurch gekennzeichnet, dass** die Probelösung und die Enzymlösung, welche eine Verdünnung von zwischen 1:60.000 und 1:90.000 aufweist, zu dem oberflächengebunden östronsulfatspezifischen Antikörper gegeben werden.

6. Verfahren nach Anspruch 5, **dadurch gekennzeichnet, dass** eine Lösung des östronsulfatspezifischen Antikörpers in einer Verdünnung von 1:90.000 bis 1:150.000 auf die zu beschichtende Oberfläche gegeben wird; und/oder
dass zum Beschichten der Oberfläche mit dem östronsulfatspezifischen Antikörper, die Lösung des östronsulfatspezifischen Antikörpers 4 bis 14 Stunden auf die zu beschichtende Oberfläche einwirkt; und/oder
dass nach Einwirkung der Lösung des östronsulfatspezifischen Antikörpers die beschichtete Oberfläche lediglich einmal mit Polysorbat 80 Lösung gewaschen wird.

7. Verfahren nach einem der Ansprüche 4 bis 6, **dadurch gekennzeichnet, dass** die Probelösung und die Enzymlösung in die beschichtete Kavität der Mikrotestplatte gegeben werden und das Reaktionsgemisch nach der Reaktion ausgegossen wird; und/oder
dass nach Reaktion zwischen östronsulfatspezifischem Antikörper, Östronsulfat und Enzym, das generierte immobilisierte Reaktionsprodukt lediglich zweimal mit Polysorbat 80 gewaschen wird; und/oder
dass das gewaschene immobilisierte Reaktionsprodukt mit einer Lösung, welche einen mittels des Enzyms aktivierbaren Farbstoff enthält, zusammengebracht wird und die Farbstoffkonzentration nach einer vorgegebenen Zeit fotometrisch bestimmt wird; und/oder
dass die fotometrische Messung der Farbreaktion zur Bestimmung der Farbstoffkonzentration in der Kavität der Mikrotestplatte durchgeführt wird.

8. Verfahren nach einem der Ansprüche 4 bis 7, **dadurch gekennzeichnet, dass** die Mikrotestplatte eine Vielzahl von Kavitäten enthält, und die entnommene Probelösung von unterschiedlichen Eiern in unterschiedliche Kavitäten gegeben werden, und auf automatisierte Weise die Östronsulfatkonzentration in den verschiedenen Eiern bestimmt wird, indem die fotometrische Messung in den unterschiedlichen Kavitäten automatisiert erfolgt und/oder der Schritt des Zusammengebens von Enzym- und Probelösung in die beschichtete Kavität automatisiert erfolgt.

9. Verfahren nach einem der Ansprüche 1 oder 2, **dadurch gekennzeichnet, dass** die Probelösung und die Konjugatlösung, welche eine Verdünnung von zwischen 1:20.000 und 1:100.000 aufweist, zu dem oberflächengebunden östronderivatspezifischen Antikörper gegeben werden.

10. Verfahren nach einem der Ansprüche 1 oder 2 sowie nach Anspruch 9, **dadurch gekennzeichnet, dass** eine Lösung des östronderivatspezifischen Antikörpers in einer Verdünnung von 1:90.000 bis 1:150.000 auf die zu beschichtende Oberfläche gegeben wird; und/oder
dass zum Beschichten der Oberfläche mit dem östronderivatspezifischen Antikörper, die Lösung des östronderivatspezifischen Antikörpers 4 bis 14 Stunden auf die zu beschichtende Oberfläche einwirkt; und/oder
dass nach Einwirkung der Lösung des östronderivatspezifischen Antikörpers die beschichtete Oberfläche lediglich einmal mit Polysorbat 80 Lösung gewaschen wird.

11. Verfahren nach einem der vorherigen Ansprüche 4 bis 8, **dadurch gekennzeichnet, dass** die Probelösung und die Enzymlösung in die beschichtete Kavität der Mikrotestplatte gegeben werden und das Reaktionsgemisch nach der Reaktion ausgegossen wird.

12. Verfahren nach Anspruch 3, **dadurch gekennzeichnet, dass** nach Reaktion zwischen östronsulfatspezifischem Antikörper, Östronsulfat und Enzym, das generierte immobilisierte Reaktionsprodukt lediglich zweimal mit Polysorbat 80 gewaschen wird.

13. Verfahren nach einem der vorherigen Ansprüche, **dadurch gekennzeichnet, dass** nach einer vorgegebenen Zeit eine fotometrische Messung zu Bestimmung der Konzentration an Östronderivat durchgeführt wird.

14. Verfahren nach Anspruch 13, **dadurch gekennzeichnet, dass** die fotometrische Messung der Farbreaktion zur Bestimmung der Farbstoffkonzentration in der Kavität der Mikrotestplatte durchgeführt wird.

15. Verfahren nach einem der Ansprüche 14 oder 15, **dadurch gekennzeichnet, dass** die Mikrotestplatte eine Vielzahl von Kavitäten enthält, und die entnommene Probelösung von unterschiedlichen Eiern in unterschiedliche Kavitäten gegeben werden, und auf automatisierte Weise die Östronderivatkonzentration in den verschiedenen Eiern bestimmt wird, indem die die fotometrische Messung in den unterschiedlichen Kavitäten automatisiert erfolgt und/oder der Schritt des Zusammengebens von Konjugat- und Probenlösung in die beschichtete Kavität automatisiert erfolgt.

## Claims

1. Method for the in-ovo sex identification of chicks, comprising the steps:
extracting a sample solution of allantoic fluid from an egg;
uniting the sample solution extracted from the egg with an oestrone-derivative-specific antibody and a conjugate solution with a conjugate binding to the antibody; and
identifying the oestrone-derivative concentration of the extracted allantoic fluid by means of the conjugate bound to the antibody
**characterized in that**
the oestrone-derivative-specific antibody is present directly bound to a surface, wherein the surface to be coated with the oestrone-derivative-specific antibody may be a membrane, glass, fiberglass, paper or polypropylene surface or a cavity of a micro-test plate, wherein
between 5 µl and 15 µl sample solution is extracted from the egg and
the sample solution is combined undiluted with the oestrone-derivative-specific antibody and the enzyme solution.

2. The method according to claim 1, **characterized in that** the conjugate contains a colloidal marker; and/or
the conjugate is selected from the following group of compounds: gold-labelled steroid-protein compound, colloidal gold, latex-labelled steroid-protein compound, colloidal latex.

3. The method according to one of the preceding claims, **characterized in that** the oestrone-derivative-specific antibody is an oestrone sulfate-specific antibody and the oestrone sulfate concentration of the extracted allantoic fluid is identified.

4. The method according to claim 3, **characterized in that** the conjugate solution is an enzyme solution containing an enzyme capable of activating an added dye, and that the identification of the oestrone sulfate concentration of the allantoic fluid extracted is carried out by photometrically identifying the concentration of the dye activated by the enzyme.

5. The method according to claim 4, **characterized in that** the sample solution and the enzyme solution, which has a dilution of between 1:60,000 and 1:90,000, are added to the surface-bound oestrone sulfate-specific antibody.

6. The method according to claim 5, **characterized in that** a solution of the oestrone sulfate-specific antibody is applied to the surface to be coated in a dilution of 1:90,000 to 1:150,000; and/or
to coat the surface with the oestrone sulfate-specific antibody, the solution of the oestrone sulfate-specific antibody acts on the surface to be coated for 4 to 14 hours; and/or
the coated surface is washed only once with polysorbate 80 solution after exposure to the solution of the oestrone sulfate-specific antibody.

7. The method according to one of claims 4 to 6, **characterized in that** the sample solution and the enzyme solution is added to the coated cavity of the micro-test plate and the reaction mixture is poured out after the reaction; and/or
the generated immobilized reaction product is washed only twice with polysorbate 80 after reaction between oestrone sulfate-specific antibody, oestrone sulfate and enzyme; and/or
the washed immobilized reaction product is combined with a solution containing a dye that can be activated by the enzyme and the dye concentration is photometrically identified after a predetermined time; and/or
the photometric measurement of the color reaction to identify the dye concentration is performed in the cavity of the micro-test plate.

8. The method according to claims 4 to 7, **characterized in that** the micro-test plate contains a plurality of cavities, and the sample solution extracted from different eggs is placed in different cavities, and the oestrone sulfate concentration in the different eggs is identified automatically by automating the photometric measurement in the different cavities and/or automating the step of combining enzyme and sample solution into the coated cavity.

9. The method according to claims 1 or 2, **characterized in that** the sample solution and the conjugate solution, which has a dilution of between 1:20,000 and 1:100,000, are added to the surface-bound oestrone-derivative-specific antibody.

10. The method according to claim 1 or 2 as well as according to claim 9, **characterized in that** a solution of the oestrone sulfate-specific antibody is applied to the surface to be coated in a dilution of 1:90,000 to 1:150,000; and/or
for coating the surface with oestrone-derivative-specific antibody, the solution of the oestrone-derivative-specific antibody acts on the surface to be coated for 4 to 14 hours; and/or
after acting of the solution of the oestrone-derivative-specific antibody, the coated surface is washed only once with polysorbate 80 solution.

11. The method according to one of claims 4 to 8, **characterized in that** the sample solution and the enzyme solution are applied into the cavity of the micro-test plate and the reaction mixture is poured out after the reaction.

12. The method according to claim 3, **characterized in that** the generated immobilized reaction product is washed only twice with polysorbate 80 after reaction between oestrone sulfate-specific antibody, oestrone sulfate, and enzyme.

13. The method according to one of the preceding claims, **characterized in that** a photometric measurement to identify the concentration of oestrone derivate is carried out after a predetermined time.

14. The method according to claim 13, **characterized in that** the photometric measurement of the color reaction is carried out for identifying the dye concentration in the cavity of the micro-test plate.

15. The method according to one of claims 14 or 15, **characterized in that** the micro-test plate contains a plurality of cavities, and the sample solution extracted from different eggs is placed in different cavities, and the oestrone-derivative concentration in the different eggs is identified automatically by automating the photometric measurement in the different cavities and/or automating the step of combining conjugate and sample solution into the coated cavity.

## Revendications

1. Procédé de sexage *in ovo* de poussins, comprenant les étapes consistant à :
prélever une solution d'échantillon de liquide allantoïque dans l'oeuf ;
mettre la solution d'échantillon prélevée dans l'oeuf en contact avec un anticorps spécifique à un dérivé d'oestrone et une solution de conjugué contenant un conjugué se liant audit anticorps ; et
déterminer la concentration en dérivé d'oestrone du liquide allantoïque prélevé à l'aide du conjugué lié à l'anticorps ; **caractérisé en ce que**
l'anticorps spécifique à un dérivé d'oestrone est directement lié à une surface, dans lequel la surface à revêtir avec l'anticorps spécifique à un dérivé d'oestrone est une surface d'intissé, de verre, de fibre de verre, de papier ou de polypropylène ou une cavité d'une microplaque de test, dans lequel entre 5 µl et 15 µl de solution d'échantillon sont prélevés dans l'oeuf et la solution d'échantillon est mise en contact, sans être diluée, avec l'anticorps spécifique à un dérivé d'oestrone et la solution enzymatique.

2. Procédé selon la revendication 1, **caractérisé en ce que** le conjugué contient un marqueur colloïdal ; et/ou **en ce que** le conjugué est choisi dans le groupe de composés ci-dessous : composé stéroïde-protéine marqué à l'or, or colloïdal, composé de stéroïde-protéine marqué au latex, latex colloïdal.

3. Procédé selon l'une quelconque des revendications précédentes, **caractérisé en ce que** l'anticorps spécifique à un dérivé d'oestrone est un anticorps spécifique au sulfate d'oestrone et la concentration en sulfate d'oestrone du liquide allantoïque prélevé est déterminée.

4. Procédé selon la revendication 3, **caractérisé en ce que** la solution de conjugué est une solution enzymatique contenant une enzyme capable d'activer un colorant ajouté, et **en ce que** la détermination de la concentration en sulfate d'oestrone du liquide allantoïque prélevé est mise en oeuvre au moyen d'une détermination photométrique de la concentration du colorant activé par l'enzyme.

5. Procédé selon la revendication 4, **caractérisé en ce que** la solution d'échantillon et la solution enzymatique présentant une dilution comprise entre 1:60 000 et 1:90 000 sont ajoutées à l'anticorps spécifique au sulfate d'oestrone et lié à une surface.

6. Procédé selon la revendication 5, **caractérisé en ce qu'**une solution de l'anticorps spécifique au sulfate d'oestrone est ajoutée à la surface à revêtir en respectant une dilution comprise entre 1:90 000 et 1:150 000 ; et/ou
**en ce que** la solution de l'anticorps spécifique au sulfate d'oestrone agit sur la surface à revêtir pendant 4 à 14 heures afin de revêtir la surface avec l'anticorps spécifique au sulfate d'oestrone ; et/ou
**en ce que** la surface revêtue n'est lavée qu'une seule fois avec une solution de polysorbate 80 après l'action de la solution de l'anticorps spécifique au sulfate d'oestrone.

7. Procédé selon l'une quelconque des revendications 4 à 6, **caractérisé en ce que** la solution d'échantillon et la solution enzymatique sont ajoutées dans la cavité revêtue de la microplaque de test et le mélange réactionnel est ôté après la réaction ; et/ou
**en ce que** le produit de réaction immobilisé généré n'est lavé que deux fois avec du polysorbate 80 après réaction entre l'anticorps spécifique au sulfate d'oestrone, le sulfate d'oestrone et l'enzyme ; et/ou
**en ce que** le produit de réaction immobilisé lavé est mis en contact avec une solution contenant un colorant pouvant être activé par l'enzyme et la concentration en colorant est déterminée de manière photométrique après un temps prédéfini ; et/ou **en ce que** la mesure photométrique de la réaction colorée permettant de déterminer la concentration en colorant est mise en oeuvre dans la cavité de la microplaque de test.

8. Procédé selon l'une quelconque des revendications 4 à 7, **caractérisé en ce que** la microplaque de test contient une pluralité de cavités, et les solutions d'échantillon prélevées de différents oeufs sont ajoutées dans différentes cavités, et la concentration en sulfate d'oestrone dans les différents oeufs est déterminée de manière automatisée en mettant en oeuvre de manière automatisée la mesure photométrique dans les différentes cavités et/ou en mettant en oeuvre de manière automatisée l'étape de mise en contact de la solution enzymatique et de la solution d'échantillon dans la cavité revêtue.

9. Procédé selon la revendication 1 ou 2, **caractérisé en ce que** la solution d'échantillon et la solution de conjugué qui présente une dilution comprise entre 1:20 000 et 1:100 000 sont ajoutées à l'anticorps spécifique à un dérivé d'oestrone et lié à une surface.

10. Procédé selon la revendication 1 ou 2 et selon la revendication 9, **caractérisé en ce qu'**une solution de l'anticorps spécifique à un dérivé d'oestrone est ajoutée à la surface à revêtir en respectant une dilution comprise entre 1:90 000 et 1:150 000 ; et/ou
**en ce que** la solution de l'anticorps spécifique à un dérivé d'oestrone agit sur la surface à revêtir pendant 4 à 14 heures afin de revêtir la surface avec l'anticorps spécifique à un dérivé d'oestrone ; et/ou
**en ce que**, après l'action de la solution de l'anticorps spécifique à un dérivé d'oestrone, la surface revêtue n'est lavée qu'une seule fois avec du polysorbate 80.

11. Procédé selon l'une quelconque des revendications 4 à 8, **caractérisé en ce que** la solution d'échantillon et la solution enzymatique sont ajoutées dans la cavité revêtue de la microplaque de test et le mélange réactionnel est ôté après la réaction.

12. Procédé selon la revendication 3, **caractérisé en ce que** le produit de réaction immobilisé généré n'est lavé que deux fois avec du polysorbate 80 après réaction entre l'anticorps spécifique au sulfate d'oestrone, le sulfate d'oestrone et l'enzyme.

13. Procédé selon l'une quelconque des revendications précédentes, **caractérisé en ce qu'**une mesure photométrique est mise en oeuvre après un temps prédéfini afin de déterminer la concentration en dérivé d'oestrone.

14. Procédé selon la revendication 13, **caractérisé en ce que** la mesure photométrique de la réaction colorée permettant de déterminer la concentration en colorant est mise en oeuvre dans la cavité de la microplaque de test.

15. Procédé selon la revendication 14 ou 15, **caractérisé en ce que** la microplaque de test contient une pluralité de cavités, et la solution d'échantillon prélevée de différents oeufs est ajoutée dans différentes cavités, et la concentration en dérivé d'oestrone dans les différents oeufs est déterminée de manière automatisée en mettant en oeuvre de manière automatisée la mesure photométrique dans les différentes cavités et/ou en mettant en oeuvre de manière automatisée l'étape de mise en contact de la solution de conjugué et de la solution d'échantillon dans la cavité revêtue.
